# EUROPÄISCHE PATENTSCHRIFT

(11) **EP 1 045 709 B1**
(45) Veröffentlichungstag und Bekanntmachung des Hinweises auf die Patenterteilung: **03.12.2003**
(21) Anmeldenummer: 98966273.9
(22) Anmeldetag: 09.12.1998
(51) Int. Cl.: A61M 5/168, A61M 5/14, B01F 5/02, A61K 9/00

(54) **VERFAHREN UND VORRICHTUNG ZUR IN-SITU-FORMULIERUNG EINER ARZNEISTOFFLÖSUNG ZUR PARENTERALEN APPLIKATION**
METHOD AND DEVICE FOR IN-SITU FORMULATION OF A MEDICINAL SOLUTION FOR PARENTERAL APPLICATION
PROCEDE ET DISPOSITIF POUR FORMULATION IN SITU D'UNE SOLUTION DE SUBSTANCE PHARMACEUTIQUE DESTINEE A UNE APPLICATION PARENTERALE

(30) Priorität: 22.12.1997 DE 19757224
(43) Veröffentlichungstag der Anmeldung: 25.10.2000
(73) Patentinhaber: Bayer Aktiengesellschaft, 51368 Leverkusen (DE)
(72) Erfinder: KÜHN, Bernd, D-51061 Köln (DE); WIESSMEIER, Georg, D-51061 Köln (DE); RUPP, Roland, D-51467 Bergisch Gladbach (DE); KRUMBACH, Bernd, D-51377 Leverkusen (DE); WEISMANTEL, Lothar, D-51065 Köln (DE); HERRMANN, Erhard, Atizapan de Zaragoza, Mexico 52930 (MX); KLEIN, Jürgen, D-51515 Kürten (DE)
(86) Internationale Anmeldenummer: EP9808014
(87) Internationale Veröffentlichungsnummer: WO99032175

(56) Entgegenhaltungen:
- EP-A- 0 650 739
- DE-B- 2 263 769
- US-A- 4 802 630
- US-A- 4 886 369

## Beschreibung

Ziel der Entwicklung von intravenös verabreichbaren Infusionslösungen ist grundsätzlich die gute Verträglichkeit der Zubereitung. Um diese zu gewährleisten, ist eine weitestgehende Angleichung der Formulierung an physiologische Gegebenheiten erforderlich, d.h. es sollte eine wäßrige Formulierung mit isotonischen (Osmolarität) und isohydrischen (pH-Wert) Eigenschaften vorliegen.

Pharmazeutische Wirkstoffe besitzen teilweise den Nachteil, daß sie aufgrund ihrer Schwerlöslichkeit, Hydrolyse- oder Oxidationsempfindlichkeit oder aufgrund ihrer Lichtempfindlichkeit schwer formulierbar oder in eine anwendungsfertige Form überführbar sind. Dies läßt sich letztlich darauf zurückführen, daß sich die Wirkstoffe unter üblichen Formulierunesbedingungen nicht im thermodynamischen Gleichgewicht befinden, d.h., daß sie entweder unter physiologischen Bedingungen ausfallen oder sich zersetzen. Dadurch ist man bei den Formulierungsmöglichkeiten und letzlich bei der Bereitstellung derartiger Wirkstoffe stark eingeschränkt.

Pharmazeutische Wirkstoffe, insbesondere die der jüngeren Generation, besitzen oft den Nachteil der geringen Löslichkeit in wäßrigem Medium bei physiologisch verträglichen pH-Werten. Dies trifft besonders für Wirkstoffe aus der Gruppe der Dihydropyridine, Anästhetika, Antibiotika, Antimykotika, Immunsuppressiva, ZNSwirksamer Mittel, Onkologika, Steroide, Barbiturate und Vitamine zu. Schwerlösliche Wirkstoffe besitzen nach der Definition der geltenden Arzneibücher eine Löslichkeit in Wasser von weniger als 1 Gewichtsprozent. Die Schwerlöslichkeit der Wirkstoffe stellt den Galeniker häufig vor das Problem, eine hinreichend gut verträgliche, wäßrige Infusionslösung bei starker Begrenzung des applizierten Infusionsvolumens zu entwickeln. Insbesondere auf Intensivstationen (ICU) erhält der Patient häufig mehrere Infusionslösungen parallel appliziert, wobei das zuträgliche Tagesvolumen von der individuellen Nierenfunktion abhängig ist. Ein Schwerpunkt der galenischen Entwicklung liegt daher in der Minimierung des Infusionsvolumens, wobei dies ein gegenläufiger Parameter zur Löslichkeit der Substanz ist. Formulierungszusätze wie Isotonisierungsmittel, Antioxidantien etc. reduzieren zudem die Lösungskapazität des Wassers. Häufig besitzt der Wirkstoff auch ein Löslichkeitsoder Stabilitätsoptimum außerhalb des physiologischen pH-Bereichs von 7,2-7,6, so daß beim optimalen pH-Wert die Formuliermöglichkeit weiter eingeschränkt wird.

Der Wirkstoff wird aufgrund der schlechten Löslichkeit in wäßrigen Medien in einem organischen oder organisch-wäßrigen Lösungsmittel oder bei stark sauren/alkalischen pH-Werten in wäßrigem oder wäßrig-organischem Medium gelöst (Wirkstoffkonzentrat). Um die Verträglichkeit zu gewährleisten muß vor der Applikation dieses Wirkstoffkonzentrat mit einem wäßrigen Medium (Verdünnungsmedium) verdünnt bzw. auf physiologisch verträgliche pH-Werte eingestellt werden. Hierbei können übersättigte Lösungen entstehen. Diese sind dadurch gekennzeichnet, daß der gelöste Wirkstoff in einer höheren Konzentration vorliegt, als dies im Lösungsmittel bei gegebener Temperatur durch Auflösung der Wirkstoffkristalle möglich ist. Solche Lösungen sind infolge der kinetischen Hemmung der Kristallisation zunächst visuell klar und praktisch frei von Teilchen. Thermodynamisch sind diese Lösungen jedoch nicht stabil. Sie führen daher im Laufe der Zeit zur Auskristallisation des Wirkstoffs und damit zur Partikelbildung. Da z.B. nach der Zubereitung solcher Lösungen in Krankenhäusern bis zu ihrer vollständigen Infusion in den Patienten oftmals relativ lange Zeiträume vergehen, die zumindest die Dauer der Infusion einschließen, kann es dabei zur Bildung von Partikeln in der Lösung kommen. In die Blutbahn injizierte Partikel können jedoch in Abhängigkeit von ihrer Größe und Form u.a. zum Gefäßverschluß und damit zu schwerwiegenden Schäden beim Patienten führen. Dieses Risiko kann dadurch herabgesetzt werden, daß entweder die Stabilität der Übersättigung auch über längere Zeit und unter allen Umgebungsbedingungen gewährleistet und dies sicher nachgewiesen ist, oder daß die Standzeit der übersättigten Lösung nach deren Herstellung minimiert ist. Die letztere Aufgabe wird durch die vorliegende Erfindung gelöst, nämlich indem die Herstellung der übersättigten Lösung aus einem Wirkstoffkonzentrat und einem Verdünnungsmedium unter Verwendung eines speziellen Mischers unmittelbar vor Applikation - am Arm des Patienten - erfolgt und bis zum Eintreten der Lösung in die Blutbahn nur Sekunden bis wenige Minuten verstreichen.

Bei der simultanen parenteralen Applikation unterschiedlicher Lösungen durch intravenöse Infusion werden bisher sogenannte Verbindungsstücke oder Y-Stücke (z.B. Firma Codan Art.-Nr. C87/2R) verwendet. Der Anschluß an andere Infusionsgeräte erfolgt z.B. über die sogenannten Luer-Lock Konnektoren nach DIN 13090 Teil 2 (Medizinische Einmalartikel: Medicalprodukte; Normen und weitere Unterlagen; Beuth Verlag 1989). Die Infusionslösungen, die Viskositäten im Bereich weniger mPas besitzen, werden aus unterschiedlichen Behältnissen dem betreffenden Verbindungsstück zugeführt und in diesem einfach durch Zusammenleiten miteinander vereinigt, bevor das Gemisch den Patienten erreicht. Eine solche Infusionsvorrichtung ist beispielsweise in DE-3228595-C2 beschrieben. Hier werden 2 Lösungen aus Infusionsflaschen mittels Schwerkraft über ein Y-Stück zusammengeführt und infundiert. Solche Einmalartikel sind in vielfältiger Formgebung handelsüblich. Der Nachteil der verfügbaren Vorrichtungen besteht darin, daß es nicht möglich ist, fluide Medien mit stark unterschiedlichen Viskositäten bei den vorliegenden geringen Flußraten (5-500 ml/h) ausreichend und schnell genug zu vermischen, da die handelsüblichen Verbindungsstücke bezüglich ihrer Funktion als Mischer nicht optimal sind. Besonders bei geringen Strömungsgeschwindigkeiten tritt z.T. ein Rückströmen der Lösungen und ein Verweilen von Lösungskomponenten in strömungsarmen Bereichen (Toträumen) auf. Dies kann zur Auskristallisation innerhalb des Applikationssystems führen. Parenteral applizierbare organische Lösungsmittel können Viskositäten von über 100 mPas besitzen (z.B. Macrogol 400). Bei der Vermischung des organischen Wirkstoffkonzentrates und des wäßrigen Verdünnungsmediums besteht die Gefahr, daß der Wirkstoff nach einer bestimmten Zeit aus der übersättigten Lösung auskristallisiert. Die Zeitspanne bis zum Einsetzen der Kristallisation nimmt mit steigendem Anteil der wäßrigen Phase ab. Gleichzeitig nimmt aber die Verträglichkeit mit steigendem Anteil der wäßrigen Phase zu.

Aus EP 0 605 739 A1 ist ein Mischer zur Mischung eines Kontrastmittelkonzetrats mit einem Verdünnungsmedium für die parenterale Applikation bekannt. Dieser aufwändig herzustellende Mischer weist einen helixförmigen Strömungsweg auf.

Die beschriebenen Probleme führen dazu, daß solche Konzentrat-Verdünnungssysteme für ein vermarktungsfähiges Produkt bisher nicht entwickelt wurden. Es werden vielmehr nur Lösungen als Darreichungsformen vermarktet, deren System nicht übersättigt ist. Dies wird z.B. erreicht durch den Zusatz von größeren Mengen und höheren Konzentrationen an organischen Lösungsmitteln (Ethanol, Macrogol, Propylenglycol etc.) oder Solubilisatoren und Tensiden (Tween, Cremophor), wodurch die lokale und systemische Verträglichkeit der Formulierung negativ beeinflußt wird.

GB-A-1472793 beschreibt solche Formulierungen für das Anästhetikum Propofol, bei denen der wäßrigen Grundlage grenzflächenaktive Substanzen und mit Wasser mischbare, nichtwäßrige Lösungsmittel zugesetzt sind. Als Grenzwerte für die lokale Verträglichkeit von organischen Lösungsmitteln in Infusionen sollte eine Konzentration von 20-30 % an organischem Medium nicht überschritten werden. Die systemische Verträglichkeit der Lösungsmittel ist substanzspezifisch unterschiedlich. Generell führen Lösungsmittel zu Venenreizungen und -entzündungen sowie zu Hämolyse. Stärker noch hämolytisch wirksam sind Tenside, die zudem anaphylaktische Schockreaktionen mit letalem Ausgang hervorrufen können. Daher muß solchen Formulierungen besonders kritisch begegnet werden und eine Entscheidung hierfür setzt eine entsprechende Nutzen-Risiko Abwägung voraus.

Eine weitere, jedoch nur eingeschränkt anwendbare Möglichkeit ist die Entwicklung von Darreichungsformen wie z.B. von Fettemulsionen oder Liposomenformulierungen, die aber wesentlich aufwendiger und damit teurer sind als konventionelle Lösungsformulierungen. Die Formulierung von Öl in Wasser Emulsionen für den Wirkstoff Propofol ist ebenfalls in GB-1 472 793 und weiterhin in DE-19 509 828-A1 beschrieben. Für weitere schwerlösliche Wirkstoffe finden sich Beispiele im US-Patent 4 168 308. Die Herstellung von wäßrigen Liposomendispersionen als Möglichkeit der Formulierung von schwerlöslichen Arzneistoffen ist in der EP-A-0 560 138 für Wirkstoffe der Dihydropyridinklasse näher ausgeführt. Hieraus wird der erhebliche technische Aufwand für diese Fornmlierungsalternative deutlich.

Zeichnen sich solche Formulierungsprobleme ab, wird die Entwicklung schwerlöslicher Wirkstoffe häufig nicht durchgeführt oder es verlängern sich deutlich die Entwicklungszeiten für eine Formulierung.

Aufgabe der Erfindung ist es, ein Verfahren bereitzustellen, mit dem es möglich ist, schwerlösliche Wirkstoffe auf einfache Weise zu verabreichen und gleichzeitig die Menge und Konzentration erforderlicher Hilfsstoffe (Lösungsmittel, Tenside etc.) zur Formulierung gegenüber konventionellen Formulierungen deutlich zu reduzieren oder ganz unnötig zu machen. Weiterhin soll eine Vorrichtung bereitgestellt werden, mit der es möglich ist, ein Wirkstoffkonzentrat in kürzester Zeit vollständig mit einem Verdünnungsmedium zu vermischen, so daß der Wirkstoff in gelöster Form in die Vene des Patienten gelangen kann. An die hierbei zu entwickelnde Mischvorrichtung werden zusätzliche Anforderungen gestellt: als Konstruktionsmaterial wird zweckmäßig ein solches gewählt werden, das sterilisierbar ist und bei dem kein Abrieb entsteht. Die Strömungswege werden zweckmäßig so gestaltet und dimensioniert, daß keine strömungsarmen Bereiche (Toträume) entstehen und die Strömungsgeschwindigkeiten auch bei geringen Flußraten eine intensive Mischung in der Mischvorrichtung erzeugen. Um eine geringe Verweilzeit der Infusionslösung zwischen Mischvorrichtung und Patienten zu gewährleisten, sollte die Mischvorrichtung in der Nähe des Infusionsortes angebracht sein. Der in der Mischvorrichtung vorherrschende Druck sollte maximal 1 bar betragen.

Gegenstand der Erfindung ist daher ein Verfahren zur in-situ-Formulierung einer Arzneistofflösung zur parenteralen Applikation, enthaltend die Schritte
- Bereitstellung von mindestens zwei fluiden Medien,
- Bereitstellung eines Mischers, der mindestens eine Mischkammer mit zwei Eingängen für die fluiden Medien und einen Ausgang aufweist,
- kontinuierliche Dosierung der fluiden Medien in Zuleitungen zu den Eingängen der Mischkammer,
- Abführung des im Mischer entstandenen Gemischs, wobei
- die Mischkammer ein Volumen von 0,2 µl bis 2 µl, vorzugsweise 0,4 µl bis 1,5 µl aufweist, und
- am Ausgang der Mischkammer eine Homogenisierblende mit einem hydraulischen Durchmesser von 1 bis 500 µm, vorzugsweise 100 bis 250 µm angeordnet ist.

Die so entstandene Arzneistofflösung befindet sich nicht im thermodynamischen Gleichgewicht. Der resultierende Gesamtvolumenstrom nach der Mischung beträgt 0,2 ml/h bis 500 ml/h, vorzugsweise 5 ml/h bis 500 ml/h.

Eine Arzneistofflösung, die sich nicht im thermodynamischen Gleichgewicht befindet, im Sinne der Erfindung, ist eine solche Lösung, die bei den Applikationsbedingungen die Neigung besitzt, unter chemischer oder physikalischer Veränderung in einen energieärmeren Zustand überzugehen. Eine solche chemische oder physikalische Veränderung kann z.B. darin bestehen, daß es zu einer Zersetzung z.B. durch Hydrolyse, Oxidation oder Photolyse oder zur Ausfällung des Wirkstoffs kommt. Die Formulierung der Arzneistofflösung durch Zusammenführen der dosierten Teilströme bei dem Verfahren der Erfindung erfolgt so, daß die entstehende Arzneistofflösung bis zum Eintritt in den menschlichen Körper keiner die Applikation beeinträchtigenden Veränderung unterliegt.

In einer weiteren bevorzugten Ausführungsform des Verfahrens der vorliegenden Erfindung handelt es sich bei den dosierten Teilströmen um mindestens eine Wirkstoffhaltige Lösung und mindestens ein wirkstofffreies Verdünnungsmedium, und die resultierende, nicht im thermodynamischen Gleichgewicht befindliche Lösung ist eine übersättigte Arzneistofflösung.

Bei der wirkstoffhaltigen Lösung handelt es sich bevorzugt um ein organisches oder organisch-wäßriges Wirkstoffkonzentrat und bei dem wirkstofffreien Verdünnungsmedium um ein wäßriges oder wäßrig-organisches Verdünnungsmedium.

Für die Formulierung der Wirkstoffkonzentrates werden geeignete wassermischbare, organische Lösungsmittel, deren Gemische oder Gemische mit Wasser verwendet, in denen sich der jeweilige Wirkstoff besonders gut löst. Bevorzugt werden als organische Lösungsmittel Macrogole verschiedener Molmasse, 1,2-Propylenglycol, Ethanol, Glycerol, Glycofurol, 2-Pyrrolidon und Glycolether. Dem Wirkstoffkonzentrat können weitere Hilfsstoffe, wie Stabilisatoren (Tenside, Komplexbildner) oder Antioxidantien, Isotonisierungsmittel, Mittel zur pH-Wert Einstellung u.a. zugesetzt werden. Es wird jedoch bevorzugt, auf den Zusatz von Stabilisatoren zu verzichten oder diese zumindest in der Menge erheblich zu reduzieren. Durch Auswahl des optimalen Lösungsmittels kann die Gesamtmenge an benötigtem organischem Lösungsmittel vorteilhaft reduziert werden. Das Wirkstoffkonzentrat soll eine Viskosität von 500 mPas nicht überschreiten und vorzugsweise im Bereich von 50 - 150 mPas liegen, um die Funktion des Mischers zu gewährleisten. Höherviskose Lösungsmittel wie Glycerin und höhermolekulare Macrogole werden daher mit niederviskosen Lösungsmitteln wie Ethanol, Wasser, 1,2-Propylenglycol o.a. gemischt.

Zur Verdünnung des Wirkstoffkonzentrates werden Verdünnungsmedien verwendet. Diese sind Wasser, physiologische Kochsalzlösung oder andere Elektrolytlösungen wie Ringerlösung, Ringer-Lactat-Lösung etc., Glucose-, Sorbitol-, Mannitol- oder andere kohlenhydrathaltige Lösungen, Volumenersatzlösungen (Dextrane und - derivate, Gelatine und -derivate, Stärkederivate), Serumderivate und -kombinationen oder wäßrig-organische Lösungsmittelmischungen mit Lösungsmittelkonzentrationen unterhalb 30 %, vorzugsweise unterhalb 10 %. Als Lösungsmittel kommen die bereits genannten wassermischbaren Lösungsmittel Macrogol verschiedener Molmasse, 1,2-Propylenglycol, Ethanol, Glycerol, Glycofurol, 2-Pyrrolidon und Glycolether in Betracht. Dem Verdünnungsmedium können weitere Hilfsstoffe, wie Stabilisatoren (Tenside, Komplexbildner) oder Antioxidantien, Isotonisierungsmittel, Mittel zur pH-Wert Einstellung u.a. zugesetzt werden. Es wird jedoch bevorzugt, auf Zusatz von Stabilisatoren zu verzichten oder diese zumindest in der Menge erheblich zu reduzieren. Die Viskosität des Verdünnungsmediums liegt bevorzugt im Bereich von 1 - 10 mPas, da hierdurch die Viskosität der Gesamtmischung erniedrigt wird und der des Blutes angenähert ist.

Im Sinne der vorliegenden Erfindung enthält die beim Mischen des Wirkstoffkonzentrates und des Verdünnungsmediums gebildete übersättigte Arzneistoff- bzw. Wirkstoff-Lösung mehr gelösten Wirkstoff als die maximale Wirkstoffmenge, die eine Lösung gleichen Volumens im thermodynamischen Gleichgewicht bei der gleichen Temperatur aufnehmen kann. Letztere ist die Löslichkeit des Wirkstoffes in der resultierenden Infusionslösung bei der gegebenen Temperatur.

Ergebnis der Mischung von Wirkstoffkonzentrat und Verdünnungsmedium ist eine Infusionslösung gewünschter Konzentration, in der der Wirkstoff in gelöster aber übersättigter Form vorliegt und über den Zeitraum von der Mischung bis zum Eintritt in die Vene nicht auskristallisiert. In der Vene findet eine rasche Verdünnung durch den Blutstrom und eine Bindung an Plasmaproteine statt, was einer Auskristallisation des Wirkstoffes dort entgegenwirkt. Die Konzentration an Lösungsmitteln in der Gesamtlösung kann auf deutlich weniger als 30 %, z.T. auf bis 7 % reduziert werden, wodurch eine gute lokale Verträglichkeit zu erwarten ist. Gleichzeitig wird das Gesamtvolumen der Infusionslösung und damit die Gesamtmenge an organischem Lösungsmittel und sonstigen Hilfsstoffen niedrig gehalten.

In einer weiteren Ausführungsform der Erfindung handelt es sich bei der wirkstoffhaltigen Lösung um ein wäßriges oder wäßrig-organisches Wirkstoffkonzentrat und bei dem wirkstofffreien Verdünnungsmedium um ein wäßriges oder wäßrig-organisches Verdünnungsmedium, deren pH-Werte so aufeinander abgestimmt werden, daß der nach der Mischung resultierende Gesamtvolumenstrom einen physiologisch verträglichen pH-Wert im Bereich von 3 bis 10, vorzugsweise 5-8 aufweist.

Diese Ausführungsform wird in solchen Fällen angewendet, in denen der Wirkstoff eine pH-Wert abhängige Löslichkeit oder Stabilität aufweist und gute Löslichkeiten oder Stabilitäten nur in unphysiologischen pH-Bereichen unterhalb pH 3 und oberhalb pH 10 vorliegen. Es wird dann ein entsprechend saures oder alkalisches wäßriges oder wäßrig-organisches Wirkstoffkonzentrat hergestellt.

Dem Wirkstoffkonzentrat können weitere Hilfsstoffe, wie Stabilisatoren (Tenside, Komplexbildner, Solubilisatoren), wassermischbare organische Lösungsmittel oder Antioxidantien, Isotonisierungsmittel, Puffermittel u.a. zugesetzt werden. Es wird jedoch bevorzugt, auf Zusatz von Stabilisatoren und organische Lösungsmittel zu verzichten oder diese zumindest in der Menge erheblich zu reduzieren.

Zur Verdünnung des sauren oder alkalischen Wirkstoffkonzentrates werden wäßrige Verdünnungsmedien verwendet, deren pH-Wert jeweils entgegengesetzt zum Wirkstoffkonzentrat eingestellt ist. Diese Verdünnungsmedien sind Wasser, physiologische Kochsalzlösung oder andere Elektrolytlösungen wie Ringerlösung, Ringer-Lactat-Lösung etc., Glucose-, Sorbitol-, Mannitol- oder andere kohlenhydrathaltige Lösungen, Volumenersatzlösungen (Dextrane und -derivate, Gelatine und -derivate, Stärkederivate), Serumderivate und -kombinationen oder wäßrig-organische Lösungsmittelmischungen mit Lösungsmittelkonzentrationen unterhalb 30 %, vorzugsweise unterhalb 10 %. Als Lösungsmittel kommen die bereits genannten wassermischbaren Lösungsmittel Macrogol verschiedener Molmasse, 1,2-Propylenglycol, Ethanol, Glycerol, Glycofurol, 2-Pyrrolidon und Glycolether in Betracht. Dem Verdünnungsmedium können weitere Hilfsstoffe, wie Stabilisatoren (Tenside, Komplexbildner) oder Antioxidantien, Isotonisierungsmittel, Puffermittel u.a. zugesetzt werden. Es wird jedoch bevorzugt, auf Zusatz von Stabilisatoren zu verzichten oder diese zumindest in der Menge erheblich zu reduzieren. Die Viskosität des Verdünnungsmediums liegt bevorzugt im Bereich von 1 - 10 mPas, da hierdurch die Viskosität der Gesamtmischung erniedrigt wird und der des Blutes angenähert ist.

Durch das Zusammenführen von Wirkstoffkonzentrat und Verdünnungsmedium wird der pH-Wert der Gesamtlösung auf physiologisch verträgliche pH-Werte im Bereich von 3 bis 10, vorzugsweise im Bereich von 5 bis 8 erhalten.

Das Verfahren der Erfindung eignet sich besonders zur Verabreichung von Wirkstoffen, die eine Löslichkeit in Wasser bei 20°C von weniger als 1 Gew.-% aufweisen.

Der Wirkstoff wird bevorzugt ausgewählt aus der Gruppe, die aus Dihydropyridinen, Anästhetika, Antibiotika, Antimykotika, Immunsuppressiva, ZNS-wirksame Mittel, Onkologica, Steroide, Barbiturate und Vitamine besteht.

Solche Wirkstoffe sind z.B. Paclitaxel, Docetaxel oder deren verwandte Substanzen oder Ciclosporin.

Zur Ausübung des Verfahrens wird bevorzugt ein totraumfreier, besonders bevorzugt ein miniaturisierter totraumfreier Mischer eingesetzt. Die Verweilzeit der Mischung in Mischer und Mischstrecke beträgt zweckmäßig weniger als **1** min, vorzugsweise weniger als 30 s. Als Mischer wird zweckmäßig ein Düsenmischer verwendet, bei dem die Teilströme jeweils durch eine Düse mit einem hydraulischen. Durchmesser zwischen 1 µm und 500 µm, vorzugsweise zwischen 100 µm und 250 µm geleitet werden, anschließend mit entgegengesetzt gerichteten Geschwindigkeitskomponenten in einer Mischkammer aufeinanderprallen und die gebildete Mischung als Gesamtvolumenstrom abgeführt wird. Der Düsendurchmesser wird zweckmäßig so gewählt, daß die Strömungsgeschwindigkeit in den Düsen 0,01 bis 15 m/s, vorzugsweise 0,01 bis 3 m/s beträgt.

Gegenstand der Erfindung ist weiterhin eine Vorrichtung zur in-situ-Formulierung einer Arzneistofflösung zur parenteralen Applikation, mit mindestens zwei Zuleitungen, in denen jeweils ein Vorratsbehälter und eine Dosiervorrichtung (4a, 4b) hintereinander geschaltet sind, wobei die Zuleitungen (2a, 2b) stromabwärts der Dosiervorrichtungen (4a, 4b) mit der Mischkammer (11) eines Mischers (3) verbunden sind, und die Mischkammer (11) außerdem mit einer Infusionsleitung (6) verbunden ist, wobei die Mischkammer (11) ein Volumen von 0,2 µl bis 2 µl, vorzugsweise 0,4 µl bis 1,5 µl aufweist, und am Ausgang der Mischkammer (11) eine Homogenisierblende (10) mit einem hydraulischen Durchmesser von 1 bis 500 µm, vorzugsweise 100 bis 250 µm angeordnet ist.

Die Düsen besitzen bevorzugt einen hydraulischen Durchmesser von 1 µm bis 500 µm, besonders bevorzugt 100 µm bis 250 µm.

Die Strömungswege in den beiden Zuleitungen einschließlich der Dosiervorrichtungen. und der Düsen sind bevorzugt symmetrisch aufgebaut.

Der Düsenmischer besteht bevorzugt aus einem spritzgußfähigen Kunststoff, besonders bevorzugt aus Polycarbonat. Bei der Anwendung der erfindungsgemäßen Vorrichtung bei lichtempfindlichen Wirkstoffen kann dem Kunststoff eine Substanz beigemischt werden, die auch bei dünnen Wandstärken des Mischers einen ausreichenden Lichtschutz gewährt. Dabei wird die Substanz zweckmäßig so ausgewählt, daß sie den Wellenlängenbereich, in dem der lichtempfindliche Wirkstoff zerstört wird, wirksam abschirmt.

Gegenstand der Erfindung ist weiterhin ein Wirkstoffverabreichungs-Set, zur Durchführung des erfindungsgemäßen Verfahrens bestehend aus einer Packung mit der erfindungsgemäßen Vorrichtung und einem Vorratsbehälter zumindest für das Wirkstoffkonzentrat. Das Wirkstoffverabreichungs-Set enthält bevorzugt zusätzlich Schlauchleitungen für die Verbindungen zur Vorrichtung (Mischer) und zu den Dosiervorrichtungen. Hinsichtlich der Wirkstoffe, der Wirkstofflconzentrate und der Verdünnungsmedien sei auf die obigen Ausführungen verwiesen.

Im folgenden wird die Erfindung an Hand von Ausführungsbeispielen und Zeichnungen näher beschrieben. Es zeigen:
- Fig. 1: ein prinzipielles Verfahrensschema zu Durchführung des erfindungsgemäßen Formulierverfahrens zur parenteralen Applikation,
- Fig.2: eine bevorzugte Ausführung eines Düsenmischers zur Vermischung und Homogenisierung eines Wirkstoffkonzentrats und eines Verdünnungsmediums,
- Fig. 3 - 5: weitere Ausführungsformen des Düsenmischers und
- Fig.6: eine Versuchsapparatur zur Durchführung der nachfolgend beschriebenen Ausführungsbeispiele.

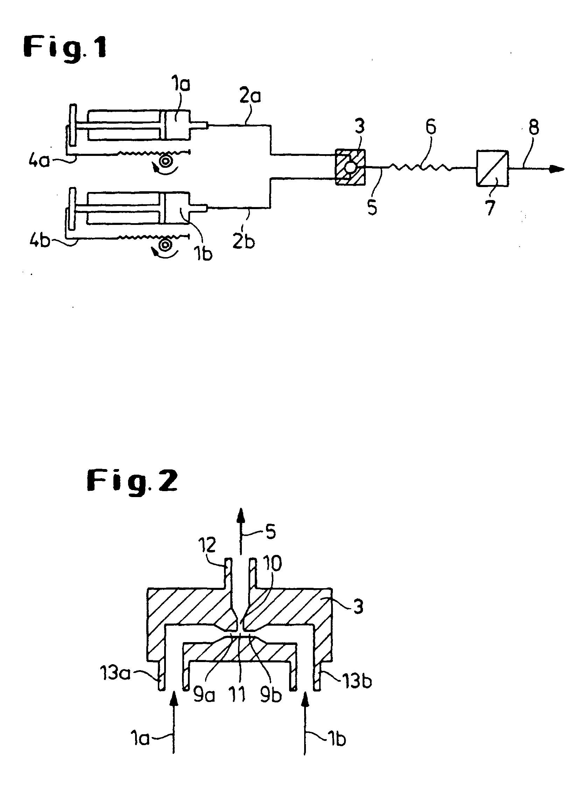

In der in Fig. 1 gezeigten Ausführungsform wird das Wirkstoffkonzentrat 1 a und das Verdünnungsmedium 1b über die Schlauchstutzen der Vorrichtung 3 mittels Spritzen- oder Infusionspumpen 4a, 4b zugeführt. Das Gemisch 5 wird unmittelbar nach dem Vermischen in der erfindungsgemäßen Vorrichtung 3 und nach Durchlaufen des Verbindungsschlauches 6 sowie ggf. eines Filters 7 und der Injektionsnadel 8 dem Patienten zugeführt.

Anstelle der Spritzen- oder Infusionspumpen können auch andere geeignete Systeme wie z.B. zwei separate Spritzen, die mit der Mischvorrichtung verbunden sind und manuell, z.B. zur Verabreichung einer Bolusinjektion, betätigt werden eingesetzt werden. In diesem Fall ist die maximale Flußrate der Teilströme und der maximale Druck im System im wesentlichen abhängig von der manuell aufgebrachten Kraft, wobei die Verträglichkeit der Applikation berücksichtigt werden muß.

Die in Fig. 2 gezeigte Ausführungsform der erfindungsgemäßen Vorrichtung besteht aus mindestens einer Düse 9a oder 9b und einer in Flußrichtung nachgeschalteten Homogenisierblende 10, deren geometrische Anordnung zueinander beliebig sein kann, einer Mischkammer 11 sowie einer Mischstrecke 12. Als Mischstrecke kann auch der Verbindungsschlauch 6 dienen. Mit Hilfe der Düsen 9a, 9b werden die jeweiligen Ausgangsfluide 1a, 1b dispergiert und der Mischkammer 11 zugeführt. Die Aufgabe der nachgeschalteten Blende 10 besteht in der Homogenisierung des resultierenden Gemischs. Die Mischstrecke 12 dient zusätzlich zur Gewährleistung einer vollständigen Vermischung bevor das Gemisch dem Patienten injiziert wird. Es werden bei der Mischung zweier Fluide (Fig. 2) zwei sich gegenüberstehende Düsen 9a, 9b verwendet; die Homogenisierblende 10 ist senkrecht und symmetrisch zu den Düsen 9a, 9b an der Mischkammer 11 angeordnet. Eine wesentliche Voraussetzung zur Lösung der geforderten Mischaufgabe ist die Minimierung des Mischkammervolumens (Mischkammer 11) zwischen Düsen 9a, 9b und Homogenisierblende 10, um den Energieeintrag je Volumen zu maximieren. Insbesondere deshalb bietet sich die miniaturisierte Bauweise der gesamten Vorrichtung an. Das Gesamt-Hohlraumvolumen beträgt beispielsweise 0.5 µl. Die Bohrungen der Düsen bzw. der Blende besitzen einen Durchmesser zwischen 0.001 und 0.5 mm, vorzugsweise 0.25 mm. Der Gesamtdruckverlust beträgt dann bei Fluidgeschwindigkeiten in den Düsen von 0.1 m/s bis 15 m/s weniger als 1 bar. Eine erfindungsgemäße Vorrichtung wird in Flachbauweise vorzugsweise aus Polycarbonat gefertigt und besitzt vorzugsweise Kantenabmessungen von 1 cm x 1 cm x 0.4 cm (L x B x H), so daß diese nach dem erfindungsgemäßen Verfahren problemlos unmittelbar in der Nähe des Injektionsortes am Patienten angebracht werden kann (Heftpflaster). Zur Stabilisierung der Vorrichtung bei der Anbringung am Arm des Patienten können zusätzlich sogenannte Flügel am Gehäuse der Vorrichtung vorgesehen werden. Für die Schlauch-Anschlüsse wurde eine in-line-Anordnung gewählt, um die mechanische Beanspruchung des Gehäuses der Vorrichtung zu vermindern. An den Schlauchenden können die entsprechenden, genormten Luer-Lock-Anschlüsse angebracht werden, über welche die erfindungsgemäße Vorrichtung an Spritzen- oder Infusionspumpen angeschlossen wird, mit denen jeweils die zu mischenden Ausgangsfluide befördert werden.

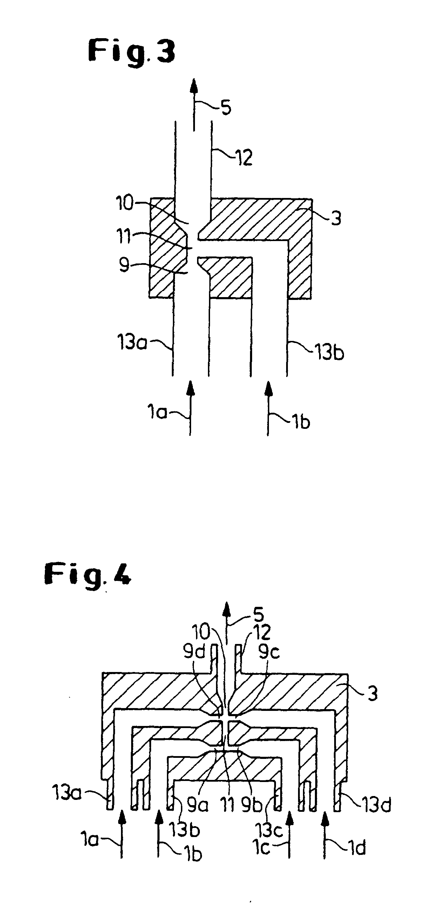

Eine weitere Ausführungsform der erfindungsgemäßen Vorrichtung, die in Fig. 3 dargestellt ist, sieht vor, daß nur ein Fluid, das niedrigviskose, durch eine Düse 9, die der Homogenisierblende 10 gegenüber angeordnet ist, zugeleitet wird; prinzipiell sind jedoch alle geometrischen Anordnungen realisierbar. So können aufgrund der miniaturisierten Bauweise beliebig viele, vorzugsweise aber weniger als 10

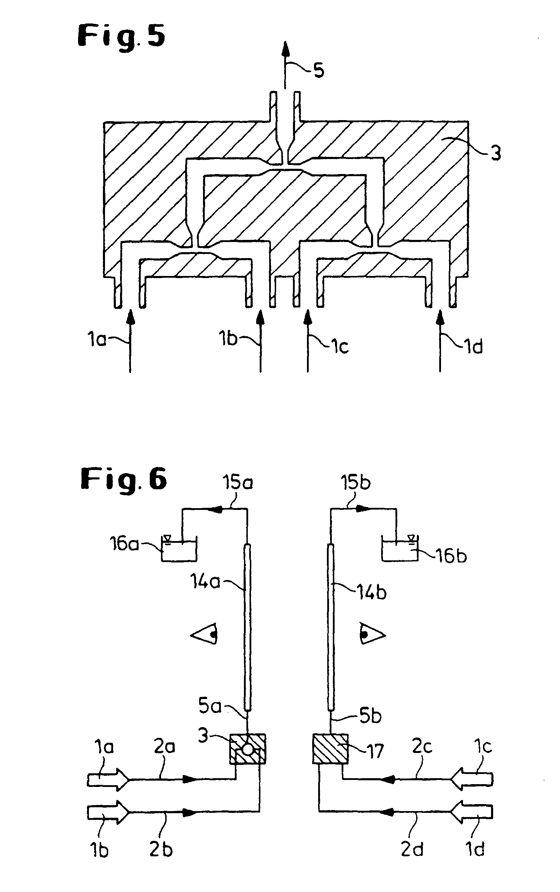

Ausgangsfluide der Vorrichtung zugeführt werden, wie dies in Fig. 4 und in Fig. 5 exemplarisch für insgesamt vier Ausgangsfluide (1a-1d) dargestellt ist. Vorzugsweise findet die Ausführungsform gemäß Fig. 5 Anwendung. Durch die hohe Integrationsdichte ist eine mehrfache Replikation der Grundstruktur in der erfindungsgemäßen Vorrichtung leicht möglich (Fig. 5).

Das erfindungsgemäße Verfahren einschließlich der erfindungsgemäßen Vorrichtung bietet den Vorteil, daß übersättigte Infusionslösungen zuverlässig und reproduzierbar hergestellt und unmittelbar nach der Herstellung appliziert werden können. Dadurch entfällt die Problematik der Stabilität der fertiggestellten Infusionslösung, die im Krankenhausbetrieb mehrere Stunden betragen muß, so daß erstmals auch intravenöse Applikationen von übersättigten Lösungen schwerlöslicher Wirkstoffe im Krankenhausbetrieb routinemäßig durchgeführt werden können. Durch die direkte Applikation ist es möglich, daß gegenüber konventionellen Lösungen eine deutlich verringerte Konzentration und Absolutmenge an die Verträglichkeit der Lösung beeinträchtigenden Lösungsmitteln erreicht wird. Außerdem besteht das Potential, auf bestimmte organische Lösungsmittel (z.B. Ethanol) in der Infusionslösung zu verzichten. Dadurch könnte der Markt für manche Infusionslösungen auf Länder ausgeweitet werden, in denen das betreffende Lösungsmittel für parenterale Applikationen nicht zugelassen ist (z.B. Japan: Ethanol). Auch auf Hilfsstoffe, die der Infusionslösung gewöhnlicherweise zur Stabilisierung zugesetzt werden, kann verzichtet oder zumindest deren Anteil deutlich reduziert werden.

Die erfindungsgemäße Vorrichtung läßt sich als Spritzgußteil realisieren, wodurch die Möglichkeit einer wirtschaftlichen Massenproduktion resultiert.

Mit Hilfe der Vorrichtung ist es möglich, Fluide mit erheblich unterschiedlicher Viskosität sehr schnell und vollständig zu vermischen. Dadurch wird vermieden, daß lokale Übersättigungen für längere Zeiträume auftreten, die eine Ausfällung des Wirkstoffes zur Folge hätten. Lokal erhöhte Konzentrationen an organischem Lösungsmittel, durch die die Blutgefäße geschädigt werden können, treten ebenfalls nicht auf.

Um die Schädigung der Blutgefäße zu vermeiden muß eine homogene Vermischung des Wirkstoffkonzentrats im Verdünnungsmedium erfolgen. Deshalb ist die Überprüfung der Vermischung ein wesentlicher Gesichtspunkt zur Charakterisierung der erfindungsgemäßen Vorrichtung.

Zum Nachweis der Funktionsfähigkeit von erfindungsgemäßem Verfahren und erfindungsgemäßer Vorrichtung wurde die im Fig. 6 im Schema dargestellte Versuchsapparatur verwendet. Dabei wurden die in der folgenden Tabelle bezeichneten verschiedenen Mischertypen verwendet:

| Mischertyp | Durchmesser | | Volumen der Mischkammer |
|---|---|---|---|
| | Düsen | Blende | |
| - | µm | µm | µl |
| A | 100 | 100 | 0.5 |
| B | 250 | 250 | 1.5 |
| C | CODAN Artikel-Nr. C87/2R | | |

Die Versuchsapparatur besteht aus zwei gleichartigen Strängen, die es ermöglichen, die erfindungsgemäße Vorrichtung (3: Mischertyp A und B) direkt mit einem konventionellen Verbindungsstück (17: Mischertyp C) zu vergleichen. Die Wirkstoffkonzentrate 1a, 1c werden mit Hilfe von Perfusorpumpen (Verwendung von 50 ml Perfusorspritzen) über die Schlauchleitungen 2a, 2c (innerer Durchmesser 2 mm) der Vorrichtung 3 und dem Verbindungsstück 17 zugeführt. Die Dosierung der Verdünnungsmedien 1b, 1d erfolgt ebenfalls über Perfusorpumpen und Schlauchleitungen 2b, 2d. Die Gemische 5a, 5b verlassen die Vorrichtung 3 bzw. das Verbindungsstück 17 und treten direkt in die Glasrohre 14a bzw. 14b (innerer Durchmesser 1.8 mm) ein. Die Gemische verlassen die Glasrohre und werden über Schläuche 15a, 15b in Auffanggefäße 16a, 16b geleitet.

Zur Beurteilung der Mischgüte wurde ein für die parenterale Applikation zugelassenes organisches Lösungsmittel mit einer Viskosität von mehr als 100 mPas (Macrogol 400), und zwar ohne Wirkstoff, verwendet. Das Lösungsmittel wurde mit einigen Tropfen HCl angesäuert, das Verdünnungsmedium Wasser (ca. 1 mPas) mit NaOH basisch gestellt und mit Phenolphthalein rot angefärbt. Die Mengenverhältnisse wurden so gewählt, daß bei dem jeweiligen Mengenverhältnis von Wasser und Lösungsmittel eine Entfärbung eintrat. Der Umschlagpunkt des verwendeten Indikators liegt zwischen pH 8 und pH 10. Die Beurteilung der Mischgüte erfolgte anhand des Entfärbungsverhaltens und der visuellen Prüfung auf gleichmäßige Phasendurchmischung, bzw. nach welcher Strecke in den Glasrohren (14) Entfärbung bzw. gleichmäßige Durchmischung festgestellt wird.

Neben der Sicherstellung einer homogenen Mischung von Wirkstoffkonzentrat und Verdünnungsmedium bestand ein weiteres Ziel darin, sicherzustellen, daß auch bei einem großen Anteil an Verdünnungsmedium in der Infusionslösung innerhalb einer bestimmten Zeit kein Wirkstoff auskristallisiert. Dazu wurden verschiedene Wirkstoffkonzentrate hergestellt, die dann auf die schon beschriebene Weise in der erfindungsgemäßen Vorrichtung 3 mit dem Verdünnungsmedium gemischt wurden. Zunächst wurde durch Beobachtung des Gemisches 5a im Glasrohr 14a festgestellt, ob sich im Gemisch sichtbare Partikel befanden (z.B. Auskristallisation aus übersättigter Lösung ). War dies nicht der Fall, wurde das Gemisch 5a unmittelbar hinter der Vorrichtung 3, die zuvor vom Glasrohr abgetrennt wurde, in ein Meßgefäß geleitet und eine Bestimmung der Partikelverteilung (HIAC) durchgeführt. Dazu wird ein Probenvolumen von 10 ml benötigt. Probennahme und Vorbereitung der Messung erfordern ca. 10 min.. Somit ist gewährleistet, daß die Standzeit der Mischung mindestens 10 min beträgt, was in einer Ausführungsform des erfindungsgemäßen Verfahrens der maximalen Zeit zwischen Mischung und Eintritt in den Patienten darstellt. Die in den Tabellen der jeweiligen Beispiele angegebenen Partikelzahlen ergaben sich aus der Mittelung von drei Messungen. Dadurch war es möglich zu überprüfen, ob die maximalen Partikelkonzentrationen gemäß den Arzneibüchern (USP XXIII: The United States Pharmacopeia 23, January 1995) nicht Arzneibüchern (USP XXIII: The United States Pharmacopeia 23, January 1995) nicht überschritten wurden. Um im gesamten Volumenstrombereich eine ausreichend gute Vermischung zu gewährleisten bietet es sich an, verschiedene Typen von Vorrichtungen zu verwenden. Die Typen unterscheiden sich im Durchmesser der Düsen und Blenden (siehe Tabelle oben). Dabei werden zunächst in einer Vorrichtung nur Düsen und Blenden mit einheitlichem Durchmesser verwendet. Je nach Mischproblem können die Durchmesser von Düsen und Blenden aber auch individuell angepaßt werden, so daß es in einer bestimmten Vorrichtung auch Düsen bzw. Blenden mit unterschiedlichen Durchmessern gibt. Im folgenden werden die verschiedenen Ausführungsbeispiele beschrieben.

### Beispiel 1: Versuche zur Beurteilung der Mischgüte .

Im Fall der erfindungsgemäßen Vorrichtungen (Mischertyp A und B) zeigte sich, daß das Gemisch (5) nach dem Austritt aus der Homogenisierblende (10) bereits entfärbt vorlag. Die gleichmäßige Durchmischung der Phasen erfolgte nach dem Durchlaufen einer Mischstrecke (11) von maximal 20 mm innerhalb von 0,4 und 17 Sekunden. Nach Ablauf dieser Verweilzeit ist eine völlige Mischung von Lösungsmittel und Verdünnungsmedium zu erwarten, wodurch Gefäßschädigungen am Patienten vermieden werden.

| Lösungsmittel | Volumenstrom | Verdünnungsmedium | Volumenstrom | Mischer-typ | Entfärbungsstrecke^{**2)**} | Misch-strecke^{**2)**} | Verweilzeit^{**3)**} |
|---|---|---|---|---|---|---|---|
| - | [ml/h] | - | [ml/h] | - | mm | mm | s |
| PEG¹⁾ | 1 | Wasser | 10 | A | 0 | ≤ 20 | ≤17,00 |
| PEG ¹⁾ | 30 | Wasser | 75 | B | 0 | ≤ 20 | ≤1,80 |
| PEG ¹⁾ | 30 | Wasser | 400 | B | 0 | ≤ 20 | ≤0,43 |
| PEG ¹⁾ | 1 | Wasser | 10 | C | ≥ 600 | ≥ 600 | ≥500,00 |
| PEG ¹⁾ | 30 | Wasser | 75 | C | ≥ 600 | ≥ 600 | ≥53,00 |
| PEG ¹⁾ | 30 | Wasser | 400 | C | ≥ 600 | ≥ 600 | ≥13,00 |

| | | | | | | | |
|---|---|---|---|---|---|---|---|
| **1)** PEG: Macrogol 400; | | | | | | | |
| **2)** Weg im Glasrohr mit einem Durchmesser von 1.8 mm (siehe Bild 6, 14a bzw. 14b); | | | | | | | |
| **3)** Verweilzeit im Glasrohr bis zur gleichmäßigen Durchmischung . | | | | | | | |

Im Fall des handelsüblichen Verbindungsstücks (Mischertyp C) konnte keine Vermischung von Verdünnungsmedium und Lösungsmittel festgestellt werden. Durch nahezu das gesamte Glasrohr (L=600 mm) verliefen zwei Fluidfäden, einer gefärbt (Verdünnungsmedium), der andere farblos (Lösungsmittel). In manchen Bereichen des Glasrohrs fand eine teilweise Verwirbelung der Fluidfäden ohne Entfärbung statt; eine vollständige Vermischung war in diesem Fall nicht zu beobachten.

### Beispiel 2: Formulierung eines Antibiotikums der Oxazolidinon-Klasse (Bay 17-1648) mit Glycofurol als 2 %iges Wirkstoffkonzentrat.

Das Antibiotikum Bay 17-1648 ((5*S*)-3-(3-Methyl-2-benzothiazolinon-6-yl)-5-(propionylaminomethyl)oxazolidin-2-on, EP 738726) erfordert eine Tagesdosis von ca. 1000 mg. Folgende Daten für die Sättigungskonzentration (M/V) bei 20°C wurden ermittelt:

| **Lösungsmittel** | **dynam. Viskosität** **[mPas]** | **Sättigungskonzentration** | **Volumen** **Lösungsmittel je** **Dosis (1000mg)** |
|---|---|---|---|
| Wasser (W) | 1 | 0,045 % (45 mg/100 ml) | 2222 ml |
| Propylenglycol (PG) | 60 | 0,7 % (700 mg/100 ml) | 143 ml |
| Macrogol 400 (PEG) | 100-130 | 1,6 % (1600 mg/l 00 ml) | 63 ml |
| Glycofurol (GF) | ca. 50 | 2,2 % (2200 mg/100 ml) | 45 ml |

Nimmt man die Sättigungskonzentrationen als Basis für die Formulierungen, so erfordert die Tagesdosis von 1000 mg ein Lösungsmittelvolumen von mindestens 2222 ml W, 143 ml PG, 63 ml PEG oder 45 ml GF. Da für eine stabile Formulierung ein Sicherheitsabstand zur Sättigungskonzentration eingehalten werden muß. um auch Kältestabilität zu gewährleisten, sind die genannten Lösungsmittelvolumina die Untergrenze für eine konventionelle Lösungsmittelformulierung.

Mit GF kann ein 2 %iges Wirkstoffkonzentrat formuliert werden, welches vor Anwendung verdünnt werden muß, um hinreichend verträglich zu sein. Die anwendungsfertige Mischung mit einer Maximalkonzentration von 20-25 % GF erfordert für die Verabreichung in der Klinik mind. 6 h physikalische Stabilität gegen Auskristallisation. Verdünnungsversuche mit wäßrigen Medien, die auf die herkömmliche Weise ohne die Anwendung von erfindungsgemäßem Verfahren und erfindungsgemäßer Vorrichtung durchgeführt wurden, zeigten folgendes Ergebnis:

| | | |
|---|---|---|
| Konzentration Lösungsmittel | 25 % GF | 20 % GF |
| Verdünnungsmedium | Wasser | 20 %ige Glucoselösung |
| Wirkstoff Sättigungskonz. in GM | ca. 200 mg/100 ml (0,2 %) | ca. 100 mg/100 ml (0,1 %) |
| Wirkstoff Konzentration in GM | 500 mg/100 ml (0,5 %) | 400 mg/100 ml (0,4 %) |
| Übersättigung in GM | ca. 2,5-fach | ca. 4-fach |
| Lösungsmittelmenge pro Dosis | 50 g/1000 mg | 50 g/1000 mg |
| Kristallisation | nach 1-2 h | nach 1 h |
| | | schlechte Verträglichkeit der 20 %igen GF-Lösung im Tierversuch |

Bei der erfindungsgemäßen Verwendung der Mischvorrichtung werden 50 ml 2 %iges Wirkstoffkonzentrat (GF) und 667 ml Wasser (Verhältnis 1:13) mittels Perfusorpumpe für das Konzentrat und Infusionspumpe für das Verdünnungsmedium über die Vorrichtung zusammengeführt, so daß ein Gesamtvolumenstrom von 430 ml/h erreicht wird. Hierdurch wird die Konzentration des organischen Lösungsmittels Glycofurol in der Gesamtmischung auf 7 % reduziert. Die Applikation der Tagesdosis von 1000 mg Bay 17-1648 erfordert hier ein Infusionsvolumen von 717 ml/Tag. Die Infusionsdauer beträgt dabei ca. 100 min je 1000 mg Bay 17-1648.

Die Sättigungslöslichkeit von Bay 17-1648 bei 7 % GF in Wasser liegt bei ca. 20 mg/100 ml. Gegenüber diesem Wert ist die anwendungsfertige Mischung mit 140 mg/100 ml etwa 7fach übersättigt. Wie die Partikelmeßwerte zeigen, ist die Formulierung mindestens 1 min stabil gegen Auskristallisation.

| | | |
|---|---|---|
| Mischertyp | - | B |
| Vordruck: Verdünnungsmedium (VM) | mbar | 100 |
| Vordruck: Wirkstoffkonzentrat (WK) | mbar | 120 |
| Lösungsmittel-Konzentration (GM) | g / 100 ml ¹⁾ | 7 |
| Wirkstoff-Konzentration im Lösungsmittel | g / 100 ml ²⁾ | 2 |
| Gesamtmenge an infundiertem Lösungsmittel | ml | 50 |
| Verdünnungsmedium | - | Wasser |
| Volumen der Gesamtmischung (GM) | ml | 717 |
| Dosierrate (Wirkstoff-Gesamtdosis) | mg / min | 1000 mg / 100 min |
| Volumenstrom GM | ml/h | 430 |
| Strömungsgeschwindigkeit: Düse WK | m / s | 0,17 |
| Strömungsgeschwindigkeit: Düse VM | m / s | 2,26 |
| Strömungsgeschwindigkeit: Blende GM | m / s | 2,43 |
| Partikelzahl ≥ 10 µm [max. 25/ml] | Partikelzahl / ml | 19 |
| Partikelzahl ≥ 25 µm [max. 3/ml] | Partikelzahl / ml | 1 |

| | | |
|---|---|---|
| **1)** Masse an Lösungsmittel bezogen auf 100 ml Gesamtlösung; | | |
| **2)** Masse an Wirkstoff bezogen auf 100 ml Lösungsmittel (Wirkstoffkonzentrat). | | |

Während bei dem Versuch der Formulierung einer konventionellen Lösungsmittelformulierung keine ausreichende Stabilität der anwendungsbereiten Mischung gegen Auskristallisation und eine unbefriedigende Verträglichkeit der Lösung erreicht werden konnte, ist erst durch die erfindungsgemäße Anwendung des Verfahrens und der Vorrichtung eine gut verträgliche Anwendung des Wirkstoffes auf parenteralem Wege möglich.

### Beispiel 3: Formulierung eines Antibiotikums der Oxazolidinon-Klasse (Bay 17-1648) mit Macrogol 400 als 1,5 %iges Wirkstoffkonzentrat.

Die Sättigungslöslichkeit des Antibiotikums Bay 17-1648 in Macrogol 400 (PEG) erlaubt analog zu Beispiel 2 die Formulierung eines 1,5 %igen Wirkstoffkonzentrates, welches ebenfalls vor Anwendung verdünnt werden muß. Die anwendungsfertige Mischung mit einer Maximalkonzentration von 30 % PEG erfordert für die Verabreichung in der Klinik mind. 6 h physikalische Stabilität gegen Auskristallisation. Verdünnungsversuche mit Wasser, die auf die herkömmliche Weise ohne die Anwendung von erfindungsgemäßem Verfahren und erfindungsgemäßer Vorrichtung durchgeführt wurden, zeigten folgendes Ergebnis:

| | | | |
|---|---|---|---|
| Konzentration Lösungsmittel | 30 % PEG | 17 % PEG | 10 % PEG |
| Verdünnungsmedium | Wasser | Wasser | Wasser |
| Wirkstoff Sättigungskonz. in GM | 150 mg/100 ml (0,15 %) | 100 mg/100 ml (0,1 %) | 80 mg/100 ml (0,008 %) |
| Wirkstoff Konzentration in GM | 450 mg/100 ml (0,45 %) | 250 mg/100 ml (0,25 %) | 150 mg/100 ml (0,15 %) |
| Übersättigung in GM | 3-fach | 2,5-fach | ca. 2-fach |
| Lösungsmittelmenge pro Dosis | 66,7 g/1000 mg | 68 g/1000 mg | 66,7 g/1000 mg |
| Kristallisation | nach 24 h | nach 1 h | nach 1 h |

Die 30 %ige Lösungsmittelkonzentration ist im Tierversuch grenzwertig bezüglich der lokalen Verträglichkeit. Niedrigere Lösungsmittelkonzentrationen zeigen deutlich geringere Stabilität gegen Auskristallisation. Aufgrund der relativ hohen Viskosität des Lösungsmittels ergab sich zudem die Schwierigkeit, bei der Mischung der Lösungskomponenten eine homogene Lösung zu erreichen.

Bei der erfindungsgemäßen Verwendung der Mischvorrichtung werden 33,5 ml 1,5 %iges Wirkstoffkonzentrat (PEG) und 430 ml Wasser (Verhältnis 1:13) mittels Perfusorpumpe für das Konzentrat und Infusionspumpe für das Verdünnungsmedium über die Mischvorrichtung zusammengeführt, so daß ein Gesamtvolumenstrom von 434 ml/h erreicht wird. Hierdurch wird die Konzentration des organischen Lösungsmittels Macrogol 400 in der Gesamtmischung auf 7 % reduziert. Die Applikation der Tagesdosis von 1000 mg Bay 17-1648 erfordert hier ein Infusionsvolumen von 2 x 463,5 ml/Tag. Die Infusionsdauer beträgt dabei 2 x 64 min für insgesamt 1000 mg Bay 17-1648.

| | | |
|---|---|---|
| Mischertyp | - | B |
| Vordruck: Verdünnungsmedium (VM) | mbar | 100 |
| Vordruck: Wirkstoffkonzentrat (WK) | mbar | 170 |
| Lösungsmittel-Konzentration (GM) | g / 100 ml ¹⁾ | 7 |
| Wirkstoff-Konzentration im Lösungsmittel | g / 100 ml ²⁾ | 1,5 |
| Gesamtmenge an infundiertem Lösungsmittel | ml | 67 |
| Verdünnungsmedium | - | Wasser |
| Volumen der Gesamtmischung (GM) | ml | 927 |
| Dosierrate (Wirkstoff-Gesamtdosis) | mg / min | 1000 mg / 128 min |
| Volumenstrom GM | ml/h | 434 |
| Strömungsgeschwindigkeit: Düse WK | m / s | 0,18 |
| Strömungsgeschwindigkeit: Düse VM | m / s | 2,28 |
| Strömungsgeschwindigkeit: Blende GM | m / s | 2,46 |
| Partikelzahl ≥ 10 µm [max. 25/ml] | Partikelzahl / ml | 19 |
| Partikelzahl ≥ 25 µm [max. 3/ml] | Partikelzahl / ml | 2 |

| | | |
|---|---|---|
| **1)** Masse an Lösungsmittel bezogen auf 100 ml Gesamtlösung; | | |
| **2)** Masse an Wirkstoff bezogen auf 100 ml Lösungsmittel (Wirkstoffkonzentrat). | | |

Die Sättigungslöslichkeit von Bay 17-1648 bei 7 % PEG in Wasser liegt bei ca. 70 mg/100 ml. Gegenüber diesem Wert ist die anwendungsfertige Mischung mit 108 mg/100 ml um den Faktor 1,5 übersättigt. Wie die Partikelmeßwerte zeigen, ist die Formulierung über mindestens 1 min stabil gegen Auskristallisation.

### Beispiel 4: Formulierung eines Antibiotikums der Oxazolidinon-Klasse (Bay 34-7780) als 5 %iges wäßriges Wirkstoffkonzentrat mit saurem pH-Wert.

Das Antibiotikum Bay 34-7780 ((5*S*)-3-[2-(3-Pyridyl)pyridin-5-yl]-5-(methoxycarbonylaminomethyl)oxazolidin-2-on-Hydrochlorid, EP-789026) weist als Hydrochlorid eine stark pH-abhängige Löslichkeit auf, welche nur die Formulierung eines sauren (pH 2,5) Wirkstoffkonzentrates erlaubt. Die Sättigungskonzentrationen sind wie folgt:

| pH-Wert | Sättigungskonzentration |
|---|---|
| 2,5 | 110 g/l |
| 4 | 0,4 g/l |

Die Formulierung einer verträglichen wäßrigen Infusionslösung mit einem pH Wert von 4,0 wäre demnach bei einer Tagesdosis von 1000 mg mit einem Infusionsvolumen von mindestens 2,5 l verbunden.

Für die angestrebte Tagesdosis von 1000 mg bei einem maximalen Infusionsvolumen von 500 ml kann nur eine Formulierung mit pH 2,5 hergestellt werden. Diese muß vor Applikation auf einen pH von mindestens 4,0 eingestellt werden. Durch Überschreiten der Sättigungskonzentration um den Faktor 5 kommt es dabei nach kurzer Standzeit zur Auskristallisation.

Der pKa-Wert des Wirkstoffs liegt bei 4,2 für eine mittelstarke Säure. Werden 50 % der Säure durch Basenzusatz neutralisiert, so stellt sich entsprechend der Henderson-Hasselbalchschen Gleichung ein pH-Wert entsprechend dem pKa-Wert von 4,2 ein.

Bei der erfindungsgemäßen Verwendung der Mischvorrichtung werden 20 ml 5 %iges wäßriges Wirkstoffkonzentrat pH 2,5 und 480 ml Natronlauge pH 10,5 (Verhältnis 1:24) mittels Perfusorpumpe für das Konzentrat und Infusionspumpe für das Verdünnungsmedium über die Mischvorrichtung zusammengeführt, so daß ein Gesamtvolumenstrom von 500 ml/h erreicht wird. Hierdurch wird der pH-Wert der Lösung auf ca. pH 4 gebracht. Die Applikation der Tagesdosis von 1000 mg Bay 34-7780 erfordert ein Infusionsvolumen von 500 ml/Tag. Die Infusionsdauer beträgt dabei 60 min für 1000 mg Bay 34-7780.

Die Sättigungslöslichkeit von Bay 34-7780 bei pH 4 liegt bei ca. 40 mg/100 ml. Gegenüber diesem Wert ist die anwendungsfertige Mischung mit 200 mg/100 ml mit dem Faktor 5 übersättigt. Durch erfindungsgemäße Verwendung der Homogenisiervorrichtung kann eine Lösung mit ausreichend verträglichem pH-Wert verabreicht werden.

| | | |
|---|---|---|
| Mischertyp | - | B |
| Vordruck: Verdünnungsmedium (VM) | mbar | 120 |
| Vordruck: Wirkstoffkonzentrat (WK) | mbar | < 100 |
| Wirkstoff-Konzentration im Lösungsmittel | g / 100 ml ¹⁾ | 5 |
| Gesamtmenge an infundiertem Lösungsmittel | ml | 20 (pH 2,5) |
| Verdünnungsmedium | - | NaOH (pH 10,5) |
| Volumen der Gesamtmischung (GM) | ml | 500 |
| Dosierrate (Wirkstoff-Gesamtdosis) | mg / min | 1000 mg / 60 min |
| Volumenstrom GM | ml/h | 500 |
| Strömungsgeschwindigkeit: Düse WK | m / s | 0,11 |
| Strömungsgeschwindigkeit: Düse VM | m / s | 2,72 |
| Strömungsgeschwindigkeit: Blende GM | m / s | 2,83 |
| Partikelzahl ≥ 10 µm [max. 25/ml] | Partikelzahl/ml | 17 |
| Partikelzahl ≥ 25 µm [max. 3/ml] | Partikelzahl/ml | 1 |

| | | |
|---|---|---|
| **1)** Masse an Wirkstoff bezogen auf 100 ml Lösungsmittel (Wirkstoffkonzentrat). | | |

### Beispiel 5: Formulierung eines Dihydropyridins (Bay y 5959, Ca-Promotor) mit Macrogol 400 als 0,5 %iges und 0,2 %iges Wirkstoffkonzentrat.

Das Dihydropyridin Bay y 5959 ((-)-(R)-Isopropyl-2-amino-5-cyano-1,4-dihydro-6-methyl-4-(3-phenyl-chinolin-5-yl)-pyridin-3-carboxylat, Ca-Promotor, EP-0 515 940 A1) ist mit ca. 0,1 µg/100 ml sehr schwer wasserlöslich, jedoch mit ca. 10 g/100 ml gut löslich in Macrogol 400 (PEG).

Eine 30 %ige PEG-Lösung mit einem Wirkstoffgehalt von 100 mg / 100 ml, die auf die herkömmliche Weise ohne die Anwendung von erfindungsgemäßem Verfahren und erfindungsgemäßer Vorrichtung angefertigt wurde, ist zwar über 12h stabil gegen Auskristallisation, die Anwendung von Bay y 5959 erfordert jedoch eine wiederholte Applikation über 5 Tage Therapiedauer. In dieser Langzeitanwendung erwies sich die 30 %ige PEG-Konzentration als schlecht venenverträglich. Daher muß die Konzentration organischer Lösungsmittel in der Applikationsmischung auf max. ca 15 % PEG beschränkt werden.

| | |
|---|---|
| Konzentration Lösungsmittel | 30 % PEG |
| Verdünnungsmedium | Wasser |
| Wirkstoff Sättigungskonz. in GM | ca. 4 mg/100 ml (0,004 %) |
| Wirkstoff Konzentration in GM | 100 mg/100 ml (0,1 %) |
| Übersättigung in GM | ca. 25-fach |
| Lösungsmittelmenge pro Dosis | 30 g/100 mg |
| Kristallisation | nach 12 h |

### Beispiel 5.1

Bei der erfindungsgemäßen Verwendung der Mischvorrichtung werden 20 ml 0,5 %iges Wirkstoffkonzentrat und 100 ml Wasser (Verhältnis 1:5) mittels Perfusorpumpe für das Konzentrat und Infusionspumpe für das Verdünnungsmedium über die Mischvorrichtung zusammengeführt, so daß ein Gesamtvolumenstrom von 180 ml/h erreicht wird. Hierdurch wird die Konzentration des organischen Lösungsmittels Macrogol 400 in der Gesamtmischung auf 16,7 % reduziert. Die Applikation der Dosis von 100 mg Bay y 5959 erfordert hier ein Infusionsvolumen von 120 ml. Die Infusionsdauer beträgt dabei 40 min für 100 mg Bay y 5959.

| | | |
|---|---|---|
| Mischertyp | - | B |
| Vordruck: Verdünnungsmedium (VM) | mbar | < 100 |
| Vordruck: Wirkstoffkonzentrat (WK) | mbar | < 100 |
| Lösungsmittel-Konzentration (GM) | g / 100 ml ¹⁾ | 16,7 |
| Wirkstoff-Konzentration im Lösungsmittel | g / 100 ml ²⁾ | 0,5 |
| Gesamtmenge an infundiertem Lösungsmittel | ml | 20 |
| Verdünnungsmedium | - | Wasser |
| Volumen der Gesamtmischung (GM) | ml | 120 |
| Dosierrate (Wirkstoff-Gesamtdosis) | mg / min | 100 mg / 40 min |
| Volumenstrom GM | ml/h | 180 |
| Strömungsgeschwindigkeit: Düse WK | m / s | 0,17 |
| Strömungsgeschwindigkeit: Düse VM | m / s | 0,85 |
| Strömungsgeschwindigkeit: Blende GM | m / s | 1,02 |
| Partikelzahl ≥ 10 µm [max 25/ml] | Partikelzahl / ml | 8 |
| Partikelzahl ≥ 25 µm [max 3/ml] | Partikelzahl / ml | 1 |

| | | |
|---|---|---|
| **1)** Masse an Lösungsmittel bezogen auf 100 ml Gesamtlösung | | |
| **2)** Masse an Wirkstoff bezogen auf 100 ml Lösungsmittel (Wirkstoffkonzentrat). | | |

### Beispiel 5.2

In einem weiteren Beispiel werden 50 ml 0,2 %iges Wirkstoffkonzentrat und 283 ml Wasser (Verhältnis 1:5,7) mittels Perfusorpumpe für das Konzentrat und Infusionspumpe für das Verdünnungsmedium über die Mischvorrichtung zusammengeführt, so daß ein Gesamtvolumenstrom von 200 ml/h erreicht wird. Hierdurch wird die Konzentration des organischen Lösungsmittels Macrogol 400 in der Gesamtmischung auf 15 % reduziert. Die Applikation der Dosis von 100 mg Bay y 5959 erfordert hier ein Infusionsvolumen von 333 ml. Die Infusionsdauer beträgt dabei 100 min für 100 mg Bay y 5959.

| | | |
|---|---|---|
| Mischertyp | - | B |
| Vordruck: Verdünnungsmedium (VM) | mbar | < 100 |
| Vordruck: Wirkstoffkonzentrat (WK) | mbar | < 100 |
| Lösungsmittel-Konzentration (GM) | g / 100 ml ¹⁾ | 15 |
| Wirkstoff-Konzentration im Lösungsmittel | g / 100 ml ²⁾ | 0,2 |
| Gesamtmenge an infundiertem Lösungsmittel | ml | 50 |
| Verdünnungsmedium | - | Wasser |
| Volumen der Gesamtmischung (GM) | ml | 333 |
| Dosierrate (Wirkstoff-Gesamtdosis) | mg / min | 100 mg /100 min |
| Volumenstrom GM | ml/h | 200 |
| Strömungsgeschwindigkeit: Düse WK | m / s | 0,17 |
| Strömungsgeschwindigkeit: Düse VM | m / s | 0,96 |
| Strömungsgeschwindigkeit: Blende GM | m / s | 1,13 |
| Partikelzahl ≥ 10 µm [max. 25/ml] | Partikelzahl / ml | 6 |
| Partikelzahl ≥ 25 µm [max. 3/ml] | Partikelzahl / ml | 1 |

| | | |
|---|---|---|
| **1)** Masse an Lösungsmittel bezogen auf 100 ml Gesamtlösung; | | |
| **2)** Masse an Wirkstoff bezogen auf 100 ml Lösungsmittel (Wirkstoffkonzentrat). | | |

Mit Bay y 5959 werden folgende Übersättigungsraten erzielt:

| Konzentration des LM (PEG) | Wirkstoff Sättigungskonzentration in GM | Wirkstoffkonzentration in GM | Übersättigung in GM | PEG-Menge pro 100 mg Wirkstoffdosis |
|---|---|---|---|---|
| 30,0 % | 0,0040 g/100 ml | 0,10 g/100 ml | 25-fach | 30 g |
| 16,7 % | ca. 0,0012 g/100 ml | 0,08 g/100 ml | 67-fach | 21 g |
| 15,0 % | ca. 0,0012 g/100 ml | 0,03 g/100 ml | 25-fach | 50 g |
| LM = Lösungsmittel; GM = Gesamtmischung | | | | |

Durch die erfindungsgemäße Verwendung der Vorrichtung lassen sich bis 67-fach übersättigte Infusionslösungen zur Anwendung bringen, ohne daß der Wirkstoff im Applikationssystem auskristallisiert. In diesem Fall ist neben der Reduktion der Lösungsmittelkonzentration unter Verbesserung der lokalen Verträglichkeit auch die deutliche Verringerung der Lösungsmittelmenge pro Dosis erreicht, wodurch die systemische Verträglichkeit gesteigert wird.

### Beispiel 6: Formulierung des Dihydropyridins Nifedipin (Adalat®) mit Macrogol 400 als 0,2 %iges Wirkstoffkonzentrat.

Nifedipin ist als i.v.-Formulierung als 0,01 %ige wäßrige Lösung mit 15 % Ethanol und 15 % Macrogol 400 (PEG) im Handel. Die Formulierung beruht auf der geringen Wasserlöslichkeit von Nifedipin. Durch die Verwendung von Ethanol und PEG ist die Verträglichkeit nicht optimal und eine Zulassung in Japan wegen des Ethanolgehaltes nicht möglich.

| | |
|---|---|
| Konzentration Lösungsmittel | 30 % (15 % PEG + 15 % Ethanol) |
| Verdünnungsmedium | Wasser |
| Wirkstoff Sättigungskonz. in GM | ca. 42 mg/100 ml (0,042 %) |
| Wirkstoff Konzentration in GM | 10 mg/100 ml (0, 1 %) |
| Übersättigung in GM | keine |
| Lösungsmittelmenge pro Dosis | 15 g/5 mg (7,5 g PEG + 7,5 g Ethanol) |
| Kristallisation | keine |

Durch die erfindungsgemäße Verwendung der Mischvorrichtung kann formulierseitig analog zu Beispiel 5 eine Lösung des Nifedipins in PEG eingesetzt und auf Ethanol verzichtet werden.

Es werden 2,5 ml 0,2 %iges Wirkstoffkonzentrat und 25,5 ml Wasser (Verhältnis 1:10) mittels zweier Perfusorpumpen über die Mischvorrichtung zusammengeführt, so daß ein Gesamtvolumenstrom von 6,6 ml/h erreicht wird. Hierdurch wird die Konzentration des organischen Lösungsmittels PEG in der Gesamtmischung auf 8,9 % reduziert. Die Applikation der Dosis von 5 mg Nifedipin erfordert ein Infusionsvolumen von 28 ml, die Infusionsdauer beträgt dabei 255 min, entsprechend einer therapeutischen Dosierrate von ca. 1,2 mg/h.

| | | |
|---|---|---|
| Mischertyp | - | A |
| Vordruck: Verdünnungsmedium (VM) | mbar | < 100 |
| Vordruck: Wirkstoffkonzentrat (WK) | mbar | < 100 |
| Lösungsmittel-Konzentration (GM) | g / 100 ml ¹⁾ | 8,9 |
| Wirkstoff-Konzentration im Lösungsmittel | g / 100 ml ²⁾ | 0,2 |
| Gesamtmenge an infundiertem Lösungsmittel | ml | 2,5 |
| Verdünnungsmedium | - | Wasser |
| Volumen der Gesamtmischung (GM) | ml | 28 |
| Dosierrate (Wirkstoff-Gesamtdosis) | mg / min | 5 mg / 255 min |
| Volumenstrom GM | ml/h | 6,6 |
| Strömungsgeschwindigkeit: Düse WK | m / s | 0,02 |
| Strömungsgeschwindigkeit: Düse VM | m / s | 0,21 |
| Strömungsgeschwindigkeit: Blende GM | m / s | 0,23 |
| Partikelzahl ≥ 10 µm [max. 25/ml] | Partikelzahl / ml | 12 |
| Partikelzahl ≥ 25 µm [max. 3/ml] | Partikelzahl / ml | 1 |

| | | |
|---|---|---|
| **1)** Masse an Lösungsmittel bezogen auf 100 ml Gesamtlösung; | | |
| **2)** Masse an Wirkstoff bezogen auf 100 ml Lösungsmittel (Wirkstoffkonzentrat). | | |

Es ist dabei möglich nach Verdünnung auf Anwendungskonzentration unter Verwendung der Vorrichtung eine 0,02 %ige Lösung, also doppelt so konzentriert wie die Handelsformulierung, zu erhalten. Ein weiterer Vorteil besteht in der Möglichkeit, eine Lösungsmittelkonzentration von 8,9 % zu realisieren; dies entspricht einer Reduzierung der Konzentration auf ein Drittel gegenüber der Handelsformulierung. Je Dosis von 5 mg Nifedipin müssen bei Verwendung der Erfindung gegenüber der Handelsware mit 7,5 g Macrogol und 7,5 g Ethanol nur 2,5 ml Macrogol 400 infundiert werden.

Anhand der Beispiele konnte gezeigt werden, daß die vorliegende Erfindung die folgenden Vorteile bietet:
1. Möglichkeit der parenteralen Verabreichung schwerlöslicher Arzneimittel in hohen Konzentrationen.
2. Möglichkeit der zuverlässigen, reproduzierbaren und sicheren Applikation übersättigter Infusionslösungen, bei denen eine hohe Neigung zur Kristallisation des Wirkstoffes besteht, durch in-situ-Formulierung.
3. Vermeidung des Stabilitätsproblems übersättigter Lösungen.
4. Vermischung von Fluiden bei extrem geringen Volumenströmen.
5. Vermischung von Fluiden mit erheblich verschiedener Viskosität bei geringen Volumenströmen.
6. Verringerung der Menge an applizierten Hilfsstoffen.
7. Gänzliche Vermeidung von Hilfsstoffen oder Verringerung deren Konzentration.
8. Verringerung des infundierten Volumens an organischen Lösungsmitteln.
9. Durch die kleinen Abmessungen der Vorrichtung der Erfindung ist die direkte Anbringung am Patienten möglich.

## Patentansprüche

1. Verfahren zur in-situ-Formulierung einer Arzneistofflösung (5) zur parenteralen Applikation, enthaltend die Schritte
- Bereitstellung von mindestens zwei fluiden Medien (1a, 1b),
- Bereitstellung eines Mischers (3), der mindestens eine Mischkammer (11) mit zwei Eingängen für die fluiden Medien (1a, 1b) und einen Ausgang aufweist,
- kontinuierliche Dosierung der fluiden Medien (1a, 1b) in Zuleitungen (2a, 2b) zu den Eingängen der Mischkammer (11),
- Abführung des im Mischer (3) entstandenen Gemischs (5),
**dadurch gekennzeichnet, dass**
- die Mischkammer (11) ein Volumen von 0,2 µl bis 2 µl, vorzugsweise 0,4 µl bis 1,5 µl aufweist, und
- dass am Ausgang der Mischkammer (11) eine Homogenisierblende (10) mit einem hydraulischen Durchmesser von 1 bis 500 µm, vorzugsweise 100 bis 250 µm angeordnet ist.

2. Verfahren nach Anspruch 1, **dadurch gekennzeichnet, daß** der resultierende Gesamtvolumenstrom des im Mischer (3) entstandenen Gemischs (5) 0,2 ml/h bis 500 ml/h, vorzugsweise 5 ml/h bis 500 ml/h beträgt.

3. Verfahren nach Anspruch 1 oder 2, **dadurch gekennzeichnet, daß** es sich bei den fluiden Medien (1a, 1b) um mindestens eine wirkstoffhaltige Lösung und mindestens ein wirkstofffreies Verdünnungsmedium handelt und daß das im Mischer (3) entstandene Gemisch (5) eine übersättigte Lösung ist.

4. Verfahren nach Anspruch 3, **dadurch gekennzeichnet, daß** es sich bei der wirkstoffhaltigen Lösung um ein Wirkstoffkonzentrat handelt und daß das Wirkstoffkonzentrat ein organisches oder organisch-wäßriges Wirkstoffkonzentrat und das Verdünnungsmedium ein wäßriges oder wäßrig-organisches Verdünnungsmedium ist.

5. Verfahren nach Anspruch 3, **dadurch gekennzeichnet, daß** es sich bei der wirkstoffhaltigen Lösung um ein wäßriges oder wäßrig-organisches Wirkstoffkonzentrat handelt und daß es sich bei dem wirkstofffreien Verdünnungsmedium um ein wäßriges oder wäßrig-organisches Verdünnungsmedium handelt, deren pH-Werte so aufeinander abgestimmt werden, daß der nach der Mischung resultierende Gesamtvolumenstrom einen physiologisch verträglichen pH-Wert im Bereich von 3 bis 10, vorzugsweise 5-8 aufweist.

6. Verfahren nach Anspruch 3 - 5, **dadurch gekennzeichnet, daß** eine wirkstoffhaltige Lösung mit einer Viskosität ≤ 500 mPa*s, vorzugsweise mit einer Viskosität im Bereich von 50 mPa*s bis 150 mPa*s und ein wäßriges wirkstofffreies Verdünnungsmedium mit einer Viskosität im Bereich von 1 mPa*s bis 10 mPa*s miteinander vermischt werden.

7. Verfahren nach Anspruch 1 - 6, **dadurch gekennzeichnet, daß** der Wirkstoff eine Löslichkeit in Wasser bei 20°C von weniger als 1 Gew.-% aufweist.

8. Verfahren nach Anspruch 1 - 7, **dadurch gekennzeichnet, daß** der Wirkstoff ausgewählt wird aus der Gruppe, die aus Dihydropyridinen, Anästhetika, Antibiotika, Antimykotika, Immunsuppressiva, ZNS-wirksame Mittel, Onkologica, Steroide, Barbiturate und Vitamine besteht.

9. Verfahren nach Anspruch 1 - 8, **dadurch gekennzeichnet, daß** als Wirkstoff Paclitaxel, Docetaxel oder deren verwandte Substanzen oder Ciclosporin verwendet wird.

10. Verfahren nach Anspruch 1 - 9, **dadurch gekennzeichnet, daß** ein totraumfreier Mischer (3) eingesetzt wird.

11. Verfahren nach Anspruch 1 - 10, **dadurch gekennzeichnet, daß** in mindestens einem der Eingänge zur Mischkammer (11) eine Düse (9, 9a, 9b, 9c, 9d) angeordnet ist.

12. Verfahren nach Anspruch 1 - 11, **dadurch gekennzeichnet, daß** in den Eingängen zur Mischkammer (11) einander gegenüberstehende Düsen (9, 9a, 9b, 9c, 9d) angeordnet sind.

13. Verfahren nach Anspruch 11 oder 12, **dadurch gekennzeichnet, daß** die Düsen (9, 9a, 9b, 9c, 9d) einen hydraulischen Durchmesser zwischen 1 µm und 500 µm, vorzugsweise zwischen 100 µm und 250 µm aufweisen.

14. Verfahren nach Anspruch 13, **dadurch gekennzeichnet, daß** der Durchmesser der Düsen (9, 9a, 9b, 9c, 9d) so gewählt wird, daß die Strömungsgeschwindigkeit in den Düsen (9, 9a, 9b, 9c, 9d) 0,01 bis 15 m/s, vorzugsweise 0,01 bis 3 m/s beträgt.

15. Vorrichtung zur in-situ-Formulierung einer Arzneistofflösung (5) zur parenteralen Applikation,
mit mindestens zwei Zuleitungen (2a, 2b), in denen jeweils ein Vorratsbehälter und eine Dosiervorrichtung (4a, 4b) hintereinander geschaltet sind, und
wobei die Zuleitungen (2a, 2b) stromabwärts der Dosiervorrichtungen (4a, 4b) mit der Mischkammer (11) eines Mischers (3) verbunden sind, und die Mischkammer (11) außerdem mit einer Infusionsleitung (6) verbunden ist,
**dadurch gekennzeichnet, daß**
die Mischkammer (11) ein Volumen von 0,2 µl bis 2 µl, vorzugsweise 0,4 µl bis 1,5 µl hat, und
daß am Ausgang der Mischkammer (11) eine Homogenisierblende (10) mit einem hydraulischen Durchmesser von 1 bis 500 µm, vorzugsweise 100 bis 250 µm angeordnet ist.

16. Vorrichtung nach Anspruch 15, **dadurch gekennzeichnet, daß** in mindestens einem der Eingänge zur Mischkammer (11) eine Düse (9, 9a, 9b, 9c, 9d) angeordnet ist.

17. Vorrichtung nach Anspruch 15 oder 16, **dadurch gekennzeichnet, daß** in den Eingängen zur Mischkammer (11) einander gegenüberstehende Düsen (9, 9a, 9b, 9c, 9d) angeordnet sind.

18. Vorrichtung nach Anspruch 16 oder 17, **dadurch gekennzeichnet, daß** die Düsen (9, 9a, 9b, 9c, 9d) einen hydraulischen Durchmesser von 1 µm bis 500 µm, vorzugsweise 100 µm bis 250 µm haben.

19. Vorrichtung nach Anspruch 17 oder 18, **dadurch gekennzeichnet, daß** die Strömungswege in den beiden Zuleitungen (2a, 2b) einschließlich der Dosiervorrichtungen (4a, 4b) und der Düsen (9, 9a, 9b, 9c, 9d) symmetrisch aufgebaut sind.

20. Vorrichtung nach Anspruch 15 - 19, **dadurch gekennzeichnet, daß** der Mischer (3) aus einem spritzgußfähigen Kunststoff, vorzugsweise aus Polycarbonat, besteht.

21. Wirkstoffverabreichungs-Set mit einer Vorrichtung nach den Ansprüchen 15 - 20 und einem Vorratsbehälter für das Wirkstoffkonzentrat.

22. Wirkstoffverabreichungs-Set nach Anspruch 21, **dadurch gekennzeichnet, daß** es zusätzlich einen Vorratsbehälter mit einem Verdünnungsmedium enthält.

23. Wirkstoffverabreichungs-Set nach Anspruch 21 - 22, **dadurch gekennzeichnet, daß** es zusätzlich Schlauchleitungen für die Verbindungen zur Vorrichtung nach den Ansprüchen 15 - 20 und zu Dosiervorrichtungen (4a, 4b) enthält.

24. Wirkstoffverabreichungs-Set nach Anspruch 21 - 23, worin der Wirkstoff eine Löslichkeit in Wasser bei 20°C von weniger als 1 Gew.-% aufweist.

25. Wirkstoffverabreichungs-Set nach Anspruch 21 - 24, worin der Wirkstoff ausgewählt wird aus der Gruppe, die aus Dihydropyridinen, Anästhetika, Antibiotika, Antimykotika, Immunsuppressiva, ZNS-wirksame Mittel, Onkologica, Steroide, Barbiturate und Vitamine besteht.

26. Wirkstoffverabreichungs-Set nach Anspruch 21, worin der Wirkstoff Paclitaxel, Docetaxel oder deren verwandte Substanzen oder Ciclosporin ist.

## Claims

1. Method for the in situ formulation of a medicinal substance solution (5) for parenteral administration, comprising the steps
- provision of at least two fluid media (1a, 1b),
- provision of a mixer (3) which has at least one mixing chamber (11) having two inlets for the fluid media (1a, 1b) and an outlet,
- continuous watering of the fluid media (1a, 1b) in feed lines (2a, 2b) to the inlets of the mixing chamber (11),
- discharging of the mixture (5) resulting in the mixer (3),
**characterized in that**
- the mixing chamber (11) has a volume of from 0.2 ml to 2 µl, pref erably 0.4 µl to 1.5 µl, and
- that a homogenizing aperture (10) with a hydraulic diameter of from 1 to 500 µm, preferably 100 to 250 µm, is disposed at the outlet from the mixing chamber (11)

2. Method according to Claim 1, **characterized in that** the resulting total volumetric flow of the mixture (5) resulting in the mixer (3) is 0.2 ml/h to 500 ml/h, preferably 5 ml/h to 500 ml/h.

3. Method according to Claim 1 or 2, **characterized in that** the fluid media (1a, 1b) comprise at least one active ingredient-containing solution and at least one active ingredient-free diluting medium, and **in that** the mixture (5) resulting in the mixer (3) is a supersaturated solution.

4. Method according to Claim 3, **characterized in that** the active ingredient-containing solution is an active ingredient concentrate, and **in that** the active ingredient concentrate is an organic or aqueous organic active ingredient concentrate and the diluting medium is an aqueous or aqueous organic diluting medium.

5. Method according to Claim 3, **characterized in that** the active ingredient-containing solution is an aqueous or aqueous organic active ingredient concentrate, and **in that** the active ingredient-free diluting medium is an aqueous or aqueous organic diluting medium, the pH values of which are matched together so that the total volumetric flow resulting after the mixing has a physiologically tolerated pH in the range from 3 to 10, preferably 5-8.

6. Method according to Claim 3 - 5, **characterized in that** an active ingredient-containing solution with a viscosity ≤ 500 mPa*s, preferably with a viscosity in the range from 50 mPa*s to 150 mPa*s and an aqueous active ingredient-free diluting medium with a viscosity in the range from 1 mPa*s to 10 mPa*s are mixed together.

7. Method according to Claim 1 - 6, **characterized in that** the active ingredient has a solubility of less than 1% by weight in water at 20°C.

8. Method according to Claim 1 - 7, **characterized in that** the active ingredient is selected from the group consisting of dihydropyridines, anaesthetics, antibiotics, antimycotics, immunosuppressants, CNS-active drugs, oncologicals, steroids, barbiturates and vitamins.

9. Method according to Claim 1 - 8, **characterized in that** paclitaxel, docetaxel or substances related thereto, or ciclosporin, is used as active ingredient.

10. Method according to Claim 1 - 9, **characterized in that** a mixer (3) without dead spaces is applied.

11. Method according to Claim 1 - 10, **characterized in that** a nozzle (9, 9a, 9b, 9c, 9d) is disposed in at least one of the inlets to the mixing chamber (11).

12. Method according to Claim 1 - 11, **characterized in that** mutually opposing nozzles (9, 9a, 9b, 9c, 9d) are disposed in the inlets to the mixing chamber (11).

13. Method according to Claim 11 or 12, **characterized in that** the nozzles (9, 9a, 9b, 9c, 9d) have a hydraulic diameter between 1 µm and 500 µm, preferably between 100 µm and 250 µm.

14. Method according to Claim 13, **characterized in that** the diameter of the nozzles (9, 9a, 9b, 9c, 9d) is chosen so that the flow velocity in the nozzles (9, 9a, 9b, 9c, 9d) is 0.01 to 15 m/s, preferably 0.01 to 3 m/s.

15. Apparatus for the in situ formulation of a medicinal substance solution (5) for parental administration, having at least two feed lines (2a, 2b), in each of which a reservoir and a metering device (4a, 4b) are connected in series, and the feed lines (2a, 2b) downstream of the metering devices (4a, 4b) to the mixing chamber (11) of a mixer (3), and the mixing chamber (11) also being connected to an infusion line (6), **characterized in that** the mixing chamber (11) has a volume of 0.2 µl to 2 µl, preferably 0.4 µl to 1.5 µl, and **in that** a homogenizing aperture (10) with a hydraluic diamter of 1 to 500 µm, preferably 100 to 250 µm, is disposed at the outlet from the mixing chamber (11).

16. Method according to Claim 15, **characterized in that** a nozzle (9, 9a, 9b, 9c, 9d) is disposed in at least one of the inlets to the mixing chamber (11).

17. Method according to Claim 15 or 16, **characterized in that** mutually opposing nozzles (9, 9a, 9b, 9c, 9d) are disposed in the inlets to the mixing chamber (11).

18. Apparatus according to Claim 16 or 17, **characterized in that** the nozzles (9, 9a, 9b, 9c, 9d) have a hydraulic diameter of 1 µm to 500 µm, preferably 100 µm to 250 µm.

19. Apparatus according to Claim 17 or 18, **characterized in that** the flow pathways in the two feed lines (2a, 2b), including the metering devices (4a, 4b) and the nozzles (9, 9a, 9b, 9c, 9d), are symmetrically designed.

20. Apparatus according to Claim 15 - 19, **characterized in that** the mixer (3) consists of an injection-mouldable plastic, preferably of polycarbonate.

21. Active ingredient administration kit with an apparatus according to Claims 15 - 20 and of a reservoir for the active ingredient concentrate.

22. Active ingredient administration kit according to Claim 21, **characterized in that** it additionally contains a reservoir with the diluting medium.

23. Active ingredient administration kit according to Claim 21 - 22, **characterized in that** it additionally contains tubing lines for the connections to the apparatus according to Claims 15 - 20 and to metering devices (4a, 4b).

24. Active ingredient administration kit according to Claim 21 - 23, in which the active ingredient has a solubility of less than 1% by weight in water at 20°C.

25. Active ingredient administration kit according to Claim 21 - 24, in which the active ingredient is selected from the group consisting of dihydropyridines, anaesthetics, antibiotics, antimycotics, immunosuppressants, CNS-active drugs, oncologicals, steroids, barbiturates and vitamins.

26. Active ingredient administration kit according to Claim 21, in which the active ingredient is paclitaxel, docetaxel or substances related thereto, or ciclosporin.

## Revendications

1. Procédé de formulation in-situ d'une solution (5) de médicament destinée à une application parentérale, qui comprend les étapes consistant :
- à préparer au moins deux milieux fluides (1a, 1b),
- à tenir prêt un mélangeur (3) qui présente au moins une chambre de mélange (11) avec deux entrées pour les milieux fluides (1a, 1b) et une sortie,
- à faire arriver en continu les milieux fluides (1a, 1b) dans des conduits (2a, 2b) aux entrées de la chambre de mélange (11),
- à décharger le mélange (5) produit dans le mélangeur (3),
**caractérisé en ce que**
- la chambre de mélange (11) présente un volume de 0,2 µl à 2 µl, de préférence de 0,4 µl à 1,5 µl, et **en ce que**
- un diaphragme d'homogénéisation (10) ayant un diamètre hydraulique de 1 à 500 µm, de préférence de 100 à 250 µm, est disposé à la sortie de la chambre de mélange (11) .

2. Procédé suivant la revendication 1, **caractérisé en ce que** le débit volumique total résultant du mélange (5) produit dans le mélangeur (3) a un débit de 0,2 ml/h à 500 ml/h, de préférence de 5 ml/h à 500 ml/h.

3. Procédé suivant la revendication 1 ou 2, **caractérisé en ce que** les milieux fluides (1a, 1b) consistent en au moins une solution contenant une substance active et au moins un milieu diluant sans substance active et **en ce que** le mélange (5) produit dans le mélangeur (3) est une solution sursaturée.

4. Procédé suivant la revendication 3, **caractérisé en ce que** la solution contenant une substance active est un concentré de substance active et **en ce que** le concentré de substance active est un concentré de substance active organique ou organique-aqueux et le milieu diluant est un milieu diluant aqueux ou aqueux-organique.

5. Procédé suivant la revendication 3, **caractérisé en ce que** la solution contenant une substance active est un concentré de substance active aqueux ou aqueux-organique et **en ce que** le milieu diluant ne contenant pas de substance active est un milieu diluant aqueux ou aqueux-organique, dont les valeurs de pH sont accordées entre elles de manière que le courant volumique total résultant après l'opération de mélange présente une valeur de pH acceptable du point de vue physiologique dans la plage de 3 à 10, de préférence de 5 à 8 .

6. Procédé suivant les revendications 3 à 5, **caractérisé en ce qu'**une solution contenant une substance active ayant une viscosité inférieure ou égale à 500 mPa*s, de préférence une viscosité comprise dans la plage de 50 mPa*s à 150 mPa*s et un milieu diluant aqueux sans substance active ayant une viscosité dans la plage de 1 mPa*s à 10 mPa*s sont mélangés ensemble.

7. Procédé suivant les revendications 1 à 6, **caractérisé en ce que** la substance active présente une solubilité dans l'eau à 20°C inférieure à 1% en poids.

8. Procédé suivant les revendications 1 à 7, **caractérisé en ce que** la substance active est choisie dans le groupe qui est constitué de dihydropyridines, d.'anesthésiques, . d'antibiotiques, d'antimycosiques, d'immunosuppresseurs, de substances agissant sur le système nerveux central, de substances pour cancérologie, de stéroïdes, de barbituriques et de vitamines.

9. Procédé suivant les revendications 1 à 8, **caractérisé en ce qu'**on utilise comme substance active le paclitaxel, le docétaxel ou des substances qui y sont apparentées, ou la ciclosporine.

10. Procédé suivant les revendications 1 à 9, **caractérisé en ce qu'**on utilise un mélangeur (3) sans espace mort.

11. Procédé suivant les revendications 1 à 10, **caractérisé en ce qu'**une buse (9, 9a, 9b, 9c, 9d) est disposée dans l'une au moins des entrées de la chambre mélangeuse (11).

12. Procédé suivant les revendications 1 à 11, **caractérisé en ce que** des buses (9, 9a, 9b, 9c, 9d) en vis-à-vis sont disposées dans les entrées de la chambre mélangeuse (11).

13. Procédé suivant la revendication 11 ou 12, **caractérisé en ce que** les buses (9, 9a, 9b, 9c, 9d) présentent un diamètre hydraulique compris entre 1 µm et 500 µm, de préférence entre 100 µm et 250 µm.

14. Procédé suivant la revendication 13, **caractérisé en ce que** le diamètre de buse (9, 9a, 9b, 9c, 9d) est choisi de manière que la vitesse d'écoulement dans les buses (9, 9a, 9b, 9c, 9d) ait une valeur de 0,01 à 15 m/s, de préférence de 0,01 à 3 m/s.

15. Dispositif pour la formulation in-situ d'une solution (5) de médicament destinée à une application parentérale,
comprenant au moins deux conduites d'admission (2a, 2b) dans chacune desquelles un réservoir et un dispositif. doseur (4a, 4b) sont montés l'un derrière l'autre, et
les conduites d'admission (2a, 2b) sont reliées en aval des dispositifs doseurs (4a, 4b) avec la chambre de mélange (11) d'un mélangeur (3), et
la chambre de mélange (11) est en outre reliée à une conduite de perfusion (6),
**caractérisé en ce que**
la chambre de mélange (11) a un volume de 0,2 µl à 2 µl, de préférence de 0,4 µl à 1,5 µl,
et **en ce que**
un diaphragme d'homogénéisation (10) ayant un diamètre hydraulique de 1 à 500 µm, de préférence de 100 à 250 µm, est disposé à la sortie de la chambre de mélange (11).

16. Dispositif suivant la revendication 15, **caractérisé en ce qu'**une buse (9, 9a, 9b, 9c, 9d) est disposée dans au moins l'une des entrées de la chambre de mélange (11).

17. Dispositif suivant la revendication 15 ou 16, **caractérisé en ce que** des buses (9, 9a, 9b, 9c, 9d) en vis-à-vis sont disposées dans les entrées de la chambre de mélange (11).

18. Dispositif suivant la revendication 16 ou 17, caractérisé en ce les buses (9, 9a, 9b, 9c, 9d) ont un diamètre hydraulique de 1 µm à 500 µm, de préférence de 100 µm à 250 µm.

19. Dispositif suivant la revendication 17 ou 18, **caractérisé en ce que** les voies d'écoulement dans les deux conduites d'admission (2a, 2b), y compris les dispositifs doseurs (4a, 4b) et les buses (9, 9a, 9b, 9c, 9d) , sont de structure symétrique.

20. Dispositif suivant les revendications 15 à 19, **caractérisé en ce que** le mélangeur (3) est réalisé en une matière synthétique apte au moulage par injection, de préférence en un polycarbonate.

21. Ensemble pour l'administration de substance active, équipé d'un dispositif suivant les revendications 15 à 20 et d'un réservoir pour le concentré de substance active.

22. Ensemble pour l'administration de substance active suivant la revendication 21, **caractérisé en ce qu'**il comporte en outre un réservoir contenant un milieu diluant.

23. Ensemble pour l'administration de substance active suivant les revendications 21 et 22, **caractérisé en ce qu'**il comporte en outre des conduites flexibles pour les composés amenés au dispositif suivant les revendications 15 à 20 et aux dispositifs doseurs (4a, 4b).

24. Ensemble pour l'administration de substance active suivant les revendications 21 à 23, dans lequel la substance active présente une solubilité dans l'eau à 20°C de moins de 1 % en poids.

25. Ensemble pour l'administration de substance active suivant les revendications 21 à 24, dans lequel la substance active est choisie dans le groupe qui est constitué de dihydropyridines, d'anesthésiques, d'antibiotiques, d'antimycosiques, d'immunosuppresseurs, de substances agissant sur le système nerveux central, de substances pour cancérologie, de stéroïdes, de barbituriques et de vitamines.

26. Ensemble pour l'administration de substance active suivant la revendication 21, dans lequel la substance active est le paclitaxel, le docétaxel ou des substances qui y sont apparentées, ou la ciclosporine.
